# EUROPÄISCHE PATENTSCHRIFT

(11) **EP 3 102 153 B1**
(45) Veröffentlichungstag und Bekanntmachung des Hinweises auf die Patenterteilung: **07.11.2018**
(21) Anmeldenummer: 15713366.1
(22) Anmeldetag: 03.02.2015
(51) Int. Cl.: A61F 2/28, A61F 2/30

(54) **VORVERBUNDENE MEHRSCHICHTIGE FOLIE ZUR ABDECKUNG EINER KNOCHENDEFEKTSTELLE**
PRE-BONDED MULTILAYER FILM FOR COVERING A BONE DEFECT SITE
FEUILLE MULTICOUCHE PRÉ-ASSEMBLÉE DESTINÉE À RECOUVRIR UN DÉFAUT OSSEUX

(30) Priorität: 05.02.2014 AT 842014
(43) Veröffentlichungstag der Anmeldung: 14.12.2016
(73) Patentinhaber: Sonnleitner, Dietmar, 5020 Salzburg (AT)
(72) Erfinder: Sonnleitner, Dietmar, 5020 Salzburg (AT)
(74) Vertreter: Torggler & Hofinger Patentanwälte
(86) Internationale Anmeldenummer: PCT/AT2015/000015
(87) Internationale Veröffentlichungsnummer: WO 2015/117170

(56) Entgegenhaltungen:
- WO-A1-00/15152
- WO-A1-2011/125760
- WO-A1-2014/086913
- WO-A2-2008/021921
- US-A1- 2013 288 199

## Beschreibung

Die Erfindung betrifft eine vorverbundene mehrschichtige Folie zur Abdeckung einer Knochendefektstelle, insbesondere im Bereich eines Kieferknochens.

Bekannte Folien oder Membranen zur Abdeckung von Knochendefektstellen (z.B. WO 2008/021921 A2, WO 00/15152 A1, WO 2011/125760 A1) werden beispielsweise im Bereich von Kieferknochen zur Kieferaugmentation eingesetzt, um einen Kieferknochen im Fall von Knochenschwund oder Knochenverlust, der bei der Extraktion eines Zahnes oder als Folge eines entzündlichen Prozesses um einen natürlichen Zahn auftreten kann, wiederaufzubauen. Derartige Folien weisen häufig eine Formstruktur aus Titan auf, die auf einer Teflonmembran angeordnet ist und die über die Knochendefektstelle angeformt wird, sodass zwischen Folie und Knochendefektstelle ein Hohlraum entsteht, in dem Knochenmaterial und bei natürlichen Zähnen auch der Zahnhalteapparat nachwachsen kann. Die Befestigung der Folie erfolgt üblicherweise mit resorbierbaren oder metallenen Pins oder Schrauben, die durch die Folie hindurch am Kieferknochen befestigt werden. Alternativ kann die Folie auch mit der Unterlage bzw. mit dem Kieferknochen verklebt werden. Bei gleichzeitig eingebrachten Implantaten kann die Folie auch am Implantatkopf befestigt werden. Da die Knochenregeneration mehrere Monate in Anspruch nimmt, ist nach erfolgtem Knochenaufbau mit einer Teflonmembran eine zweite Operation nötig, um die Folie bzw. Teflonmembran wieder aus dem Körper zu entfernen.

Aufgabe der Erfindung ist es, die vorbeschriebenen Nachteile zu vermeiden und eine gegenüber dem Stand der Technik verbesserte vorverbundene mehrschichtige Folie anzugeben. Insbesondere soll eine weitere Operation zum Entfernen der Folie vermieden werden.

Diese Aufgabe wird erfindungsgemäß durch die Merkmale des Anspruchs 1 gelöst. Vorteilhafte Ausgestaltungen der Erfindung sind in den abhängigen Ansprüchen angegeben.

Gemäß der Erfindung ist also vorgesehen, dass die Folie eine formgebende Formschicht zur Anformung der Folie an die Knochendefektstelle und wenigstens eine Deckschicht zur Abdeckung der Knochendefektstelle umfasst, wobei die Formschicht und die wenigstens eine Deckschicht im Wesentlichen vollständig resorbierbar sind.

Dadurch, dass somit die Folie oder Membran insgesamt vollständig im Körper resorbierbar ist, indem sie beispielsweise durch Hydrolyse im Körper abgebaut wird, entfällt das Durchführen einer weiteren Operation zur Entfernung der Folie. Mit anderen Worten ist hierbei lediglich eine Operation zur Anbringung der Folie erforderlich.

Die Folie umfasst eine formgebende Formschicht, die der Anformung der Folie an die Knochendefektstelle dient und durch die ein Hohlraum zwischen Knochendefektstelle und Folie gebildet werden kann, sodass in diesem Hohlraum Knochenwachstum stattfinden kann. Für eine günstige Knochenregeneration kann der Hohlraum auch Knochenersatzmaterialien und/oder Träger für Medikamente, Wachstumsfaktoren und/oder sonstige die Heilung und Knochenbildung fördernde und schützende Substanzen enthalten. Der Hohlraum wird durch die raumbildende und raumhaltende Formschicht solange aufrecht erhalten, bis der Hohlraum durch nachwachsendes Knochenmaterial ausgefüllt ist.

Darüber hinaus umfasst die Folie wenigstens eine Deckschicht zur Abdeckung der Knochendefektstelle. Diese Deckschicht, die beispielsweise als Membran ausgebildet sein kann, dient zur Überdeckung und Abdichtung der Knochendefektstelle, um das Eindringen von Weichgewebe in die Knochendefektstelle zu vermeiden. Um die Anbringung der Folie und die Abdichtung der Knochendefektstelle weiter zu verbessern, kann die wenigstens eine Deckschicht auch so ausgebildet sein, dass sie mit einem die Knochendefektstelle umgebenden Zahnfleisch verklebt.

Dadurch, dass die formgebende Formschicht und die wenigstens eine Deckschicht als bereits vorverbundene mehrschichtige Folie bereitgestellt wird, ergibt sich eine leicht handhabbare Folie zur Abdeckung einer Knochendefektstelle, welche die raumbildenden Eigenschaften der Formschicht mit den abdichtenden Eigenschaften der Deckschicht verbindet und die darüber hinaus im Körper im Wesentlichen vollständig resorbiert wird. Die einzelnen Schichten der vorgeschlagenen Folie (Formschicht und wenigstens eine Deckschicht) können mechanisch und/oder chemisch miteinander verbunden sein. Es kann also vorzugsweise vorgesehen sein, dass die Formschicht und die wenigstens eine Deckschicht mechanisch und/oder chemisch miteinander verbunden sind.

Gemäß einer bevorzugten Ausführungsform kann vorgesehen sein, dass die Formschicht und die wenigstens eine Deckschicht in unterschiedlichen Zeiträumen im Wesentlichen vollständig resorbierbar sind. So kann beispielsweise durch Auslegung der Formschicht und der wenigstens einen Deckschicht erreicht werden, dass die Formschicht schneller resorbiert als die wenigstens eine Deckschicht. Generell ergeben sich durch unterschiedlich starke Resorbierbarkeiten der Formschicht und der wenigstens eine Deckschicht große Freiheiten in der Auslegung der Folie in Bezug auf ihre Resorbierbarkeit.

Es kann vorgesehen sein, dass die Folie insgesamt in einem Zeitraum von etwa 3 bis 12 Monaten, vorzugsweise etwa 4 bis 6 Monaten, im Wesentlichen vollständig resorbierbar ist. Dies ist der Zeitraum innerhalb dessen im Normalfall der Knochenwiederaufbau erfolgt ist.

Um eine gute Anformung an die Knochendefektstelle und eine stabile Hohlraumbildung zwischen Folie und Knochendefektstelle zu ermöglichen, kann vorgesehen sein, dass die Formschicht steifer als die wenigstens eine Deckschicht ausgebildet ist. Die höhere Steifigkeit der Formschicht dient dabei dazu, einen Hohlraum für den Knochenaufbau zu bilden und diesen Hohlraum auch für den für die Knochenregeneration benötigten Zeitraum aufrecht zu erhalten. Durch eine im Vergleich zur Formschicht geringeren Steifigkeit der wenigstens einen Deckschicht kann wiederum eine gute Überdeckung und Abdichtung der Knochendefektstelle erzielt werden.

Vorzugsweise kann vorgesehen sein, dass die Formschicht, gegebenenfalls zusammen mit der wenigstens einen Deckschicht, mechanisch und/oder thermisch und/oder chemisch verformbar ausgebildet ist. So kann insbesondere die Formschicht als im Wesentlichen formstabile Schicht ausgebildet sein, die sich unter mechanischem, thermischem oder chemischem Einfluss verformen lässt und nach dieser Verformung wiederum eine ausreichende Formstabilität aufweist, um den für das Knochenwachstum zu bildenden Hohlraum für die erforderliche Zeitspanne aufrecht zu erhalten. Die wenigstens eine Deckschicht kann flexibel und vorzugsweise elastisch sein, um eine gute Abdeckung und Abdichtung der Knochendefektstelle zu ermöglichen.

Eine mechanische Verformung kann dabei beispielsweise durch Verbiegen mit einer Zange erfolgen. Dies ist insbesondere für verhältnismäßig dünne Formschichten (z.B. im Bereich von etwa 0,10 mm bis etwa 0,5 mm) eine geeignete Methode der Anformung. Für dickere Formschichten (z.B. dicker als etwa 0,5 mm) kann eine thermische Verformung einer Formschicht zur Anformung zweckmäßig sein. Eine entsprechende thermische Verformung kann dabei beispielsweise mittels eines Thermostabes mit einer heißen Spitze oder Fläche, über erwärmte vorgefertigte Modelle oder im heißen Wasserbad mit steriler Kochsalzlösung erzielt werden.

Für eine gute Resorbierbarkeit der vorgeschlagenen Folie ist erfindungsgemäss vorgesehen, dass die wenigstens eine Deckschicht im Wesentlichen vollständig, aus einem bioresorbierbaren Collagenmaterial besteht. Dabei kann vorgesehen sein, dass das bioresorbierbare Collagenmaterial Typ-I-Collagen und/oder Typ-III-Collagen umfasst. Das Collagenmaterial kann beispielsweise aus bovinen Achillessehnen stammen.

Für eine gute Resorbierbarkeit der vorgeschlagenen Folie ist erfindungsgemäss vorgesehen, dass die Formschicht zumindest teilweise, vorzugsweise im Wesentlichen vollständig, aus einem bioresorbierbaren Polymermaterial besteht. Bei dem bioresorbierbaren Polymermaterial kann es sich auch um ein Co-Polymermaterial handeln.

Eine besondere Ausführungsvariante sieht vor, dass das bioresorbierbare Polymermaterial Milchsäure, vorzugsweise L-Milchsäure, und/oder deren Derivate umfasst. Vorteilhaft ist es dabei, wenn der Anteil der Milchsäure im bioresorbierbaren Polymermaterial mindestens 70%, vorzugsweise etwa 80% bis 95%, besonders bevorzugt im Wesentlichen etwa 82%, beträgt.

Darüber hinaus kann vorgesehen sein, dass das bioresorbierbare Polymermaterial Glykolsäure umfasst. Vorteilhaft ist es dabei, wenn der Anteil der Glykolsäure im bioresorbierbaren Polymermaterial höchstens 30%, vorzugsweise etwa 15% bis 20%, besonders bevorzugt im Wesentlichen etwa 18%, beträgt. Je nach Zusammensetzung der Formschicht kann erreicht werden, dass die Formschicht im Wesentlichen formstabil und dennoch im Wesentlichen vollständig resorbierbar ist.

Bei einer weiteren bevorzugten Ausführungsform kann vorgesehen sein, dass die Formschicht und die wenigstens eine Deckschicht unterschiedliche Flächenausdehnungen aufweisen. Dabei kann vorgesehen sein, dass die Formschicht eine geringere Flächenausdehnung als die wenigstens eine Deckschicht einnimmt. Wenn die wenigstens eine Deckschicht die Formschicht aufgrund ihrer geringeren Flächenausdehnung überdeckt, kann eine besonders gute Abdeckung und somit auch Abdichtung der Knochendefektstelle erreicht werden.

Vorzugsweise kann vorgesehen sein, dass die wenigstens eine Deckschicht und/oder die Formschicht im Wesentlichen durchgehend flächig ausgebildet ist bzw. sind. Eine für die Anformung an die Knochendefektstelle günstige Kontur der Folie kann dabei beispielsweise durch entsprechendes Schneiden der Folie erreicht werden.

Besonders günstig ist es jedoch, wenn die Formschicht zur Anformung an die Knochendefektstelle eine Formstruktur aufweist. Dabei kann vorgesehen sein, dass die Formstruktur wenigstens abschnittsweise einen konvex und/oder konkav gekrümmten Rand und/oder wenigstens abschnittsweise eine konvex und/oder konkav gekrümmte Form aufweist. Mit anderen Worten kann die Formstruktur beispielsweise flächige - konvex und/oder konkav gekrümmte - Vorsprünge aufweisen und somit einen konvex und/oder konkav gekrümmten Rand aufweisen. Alternativ oder zusätzlich kann auch die Formstruktur als ganzes eine entsprechend konvex und/oder konkav gekrümmte Form aufweisen.

Besonders vorteilhaft ist es, wenn die Formstruktur wenigstens ein strebenförmiges Anformelement aufweist. Die strebenförmigen oder lappenförmigen Anformelemente können dabei bügelartig über die Knochendefektstelle geformt werden und eine beliebige Hohlraumform ermöglichen.

Besonders vorteilhaft ist jene Ausführungsform der Erfindung, bei der die Formstruktur im Wesentlichen gitterförmig ausgebildet ist. Die gitterförmige Formstruktur bildet in diesem Fall ein Verstärkungsgitter, das die Bildung einer Vielzahl von beliebigen Hohlraumformen ermöglicht.

Es kann auch vorgesehen sein, dass die Formstruktur durch wenigstens eine Verstärkung der Formschicht ausgebildet ist. Insbesondere, wenn die Formschicht in Form einer aushärtenden Flüssigkeit oder eines aushärtenden Gels auf die wenigstens eine Deckschicht aufgebracht wird, ist es günstig, wenn die Formstruktur lediglich durch das Aufbringen von mehr Flüssigkeit oder Gel im Bereich der Formstruktur erzielt werden kann. In diesem Fall kann beispielsweise die Formschicht unterschiedliche Dicken aufweisen.

Eine besondere Ausführungsvariante sieht vor, dass die Folie eine Trägerschicht für wenigstens eine darauf anzuordnende oder angeordnete Substanz aufweist. Bei den auf der Trägerschicht anzuordnenden oder angeordneten Substanzen kann es sich dabei um Medikamente, Wachstumsfaktoren und/oder sonstige die Heilung und Knochenbildung fördernde und schützende Substanzen handeln. Die Trägerschicht kann vorzugsweise an einer der Knochendefektstelle zuzuwendenden Seite der Folie angeordnet sein und zumindest teilweise, vorzugsweise im Wesentlichen vollständig, aus einem bioresorbierbaren Collagenmaterial bestehen.

Es kann auch vorgesehen sein, dass entsprechende Substanzen direkt auf der Formschicht und/oder der wenigstens einen Deckschicht angebracht sind. Es kann auch vorgesehen sein, dass die einer Knochendefektstelle zuzuwendende Seite bzw. Oberfläche der Folie selbst als Träger für die oben beschriebenen Substanzen dient, indem beispielsweise diese Seite bzw. Oberfläche der Folie eine entsprechende Rauigkeit aufweist.

Die vorgeschlagene Folie oder Membran kann je nach Anwendungsfall auch vorgeschnitten und/oder vorgeformt bereitgestellt werden. Dabei kann beispielsweise ein gewünschter Zuschnitt und/oder eine gewünschte 3D-Verformung der Folie nach einer datenverarbeitungsgestützten Planung erfolgen.

Weitere Einzelheiten und Vorteile der vorliegenden Erfindung werden anhand der nachfolgenden Figurenbeschreibung erläutert. Dabei zeigen:
- Fig. 1:: ein Ausführungsbeispiel der vorgeschlagenen vorverbundenen mehrschichtigen Folie in einer perspektivischen Explosionsdarstellung,
- Fig. 2:: eine Seitenansicht der vorgeschlagenen vorverbundenen mehrschichtigen Folie gemäß Figur 1,
- Fig. 3 - 7:: Draufsichten auf verschiedene Ausführungsformen der vorgeschlagenen vorverbundenen mehrschichtigen Folie,
- Fig. 8 - 15:: mehrere Ausführungsbeispiele der vorgeschlagenen vorverbundenen mehrschichtigen Folie in perspektivischen Explosionsdarstellungen,
- Fig. 16:: eine vorgeschlagene vorverbundene mehrschichtige Folie angeordnet an einer Knochendefektstelle eines Kieferknochens,
- Fig. 17:: eine vorgeschlagene vorverbundene mehrschichtige Folie angeordnet an einer Knochendefektstelle eines Kieferknochens mit Implantat,
- Fig. 18:: eine vorgeschlagene vorverbundene mehrschichtige Folie angeordnet an einer Knochendefektstelle eines Kieferknochens mit Implantat, Beilagscheibe und Pfosten,
- Fig. 19:: eine vorgeschlagene vorverbundene mehrschichtige Folie eingeklemmt in einer Nut einer Beilagscheibe,
- Fig. 20:: eine vorgeschlagene vorverbundene mehrschichtige Folie eingeklemmt in einer Nut einer Beilagscheibe und angeordnet an einer Knochendefektstelle eines Kieferknochens mit Implantat und Pfosten,
- Fig. 21:: eine vorgeschlagene vorverbundene mehrschichtige Folie angeordnet an einer Knochendefektstelle um einen natürlichen Zahn und
- Fig. 22:: eine vorgeschlagene vorverbundene mehrschichtige Folie angeordnet an einer Knochendefektstelle um einen natürlichen Zahn in einer Schnittdarstellung.

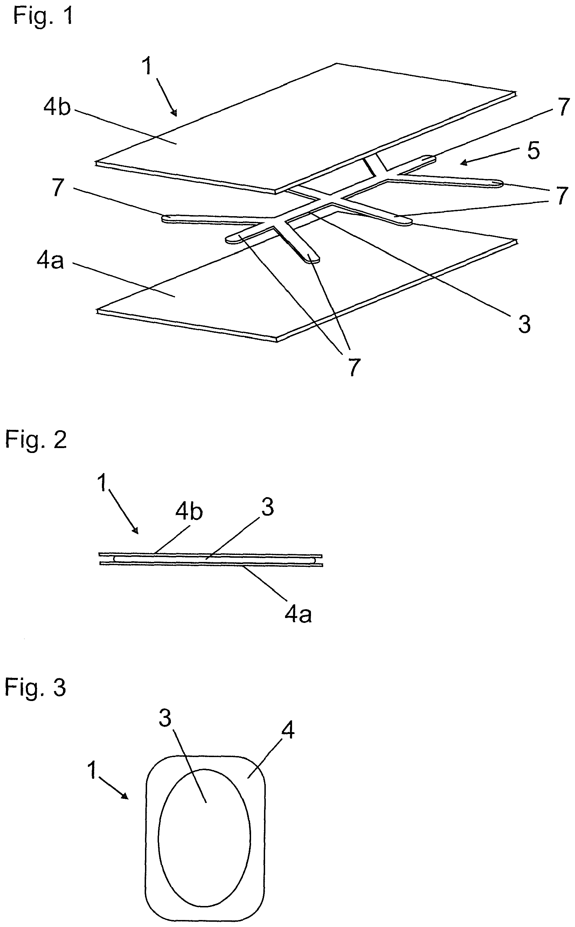

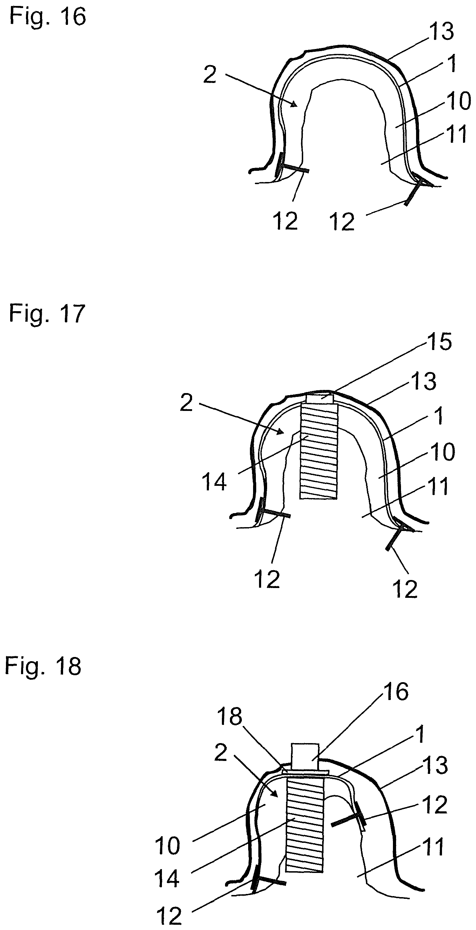

Figur 1 zeigt eine perspektivische Explosionsdarstellung einer vorgeschlagenen vorverbundenen mehrschichtigen Folie 1. Die Folie 1 umfasst eine Formschicht 3 sowie zwei Deckschichten 4a und 4b. Die Formschicht 3 ist steifer als die Deckschichten 4a und 4b ausgebildet und weist eine Formstruktur 5 auf. Die Formstruktur 5 umfasst mehrere strebenförmige Anformelemente 7, die dazu dienen, die Folie 1 über eine Knochendefektstelle 2 (hier nicht gezeigt) zu formen, wobei sich die Folie 1 durch die Anformelemente 7 gut an einen noch vorhandenen Knochen 11 der Knochendefektstelle 2 (siehe z.B. Figur 16) anformen lässt. Die Formstruktur 5 ist insgesamt im Wesentlichen gitterförmig ausgebildet und ermöglicht somit die Ausbildung beliebiger Oberflächenformen der Folie 1, sodass sich in Verbindung mit einer Knochendefektstelle 2 beliebige Hohlraumformen zwischen Folie 1 und Knochendefektstelle 2 bilden lassen.

Die Formschicht 3 sowie die Deckschichten 4a und 4b bestehen jeweils aus einem bioresorbierbaren Material, sodass die Folie 1 insgesamt im Körper im Wesentlichen vollständig resorbierbar ist. Durch das Vorsehen von zwei Deckschichten 4a und 4b, zwischen denen die Formschicht 3 eingebettet ist, lässt sich insbesondere die Resorptionsgeschwindigkeit und mechanische Festigkeit der Formschicht 3 steuern.

Bei den Deckschichten 4a und 4b handelt es sich um bioresorbierbare Collagen-Membranen, die einerseits durch ihre Weichheit eine Knochendefektstelle 2 gut abdecken können und andererseits mit einem die Knochendefektstelle 2 umgebenden Zahnfleisch 13 gut verkleben können, sodass sich eine gute Abdichtung der Knochendefektstelle 2 ergibt.

Die Formschicht 3 kann beispielsweise aus einem bioresorbierbaren Polymermaterial oder Co-Polymermaterial bestehen. Insbesondere kann die Formschicht 3 beispielsweise etwa 82% L-Milchsäure und etwa 18% Glycolsäure umfassen. Eine solche Materialwahl ergibt eine im Wesentlichen formstabile Formschicht 3, die zur Anformung an eine Knochendefektstelle 2 mechanisch, thermisch und/oder chemisch verformbar ausgebildet sein kann, wobei die Formschicht 3 nach einer solchen Verformung im Wesentlichen wiederum formstabil ist. Aufgrund der Steifigkeit und Formstabilität der Formschicht 3 kann somit zwischen der Folie 1 und einer Knochendefektstelle 2 ein Hohlraum für die Knochenregeneration geschaffen und für den Zeitraum der Knochenregeneration auch gehalten werden.

Figur 2 zeigt eine Seitenansicht der vorverbundenen mehrschichtigen Folie 1 gemäß Figur 1.

Figur 3 zeigt eine Draufsicht auf eine weitere Variante der vorgeschlagenen Folie 1, die in diesem Beispiel zweischichtig ausgeführt ist und eine Formschicht 3 und eine Deckschicht 4 umfasst. Sowohl die Formschicht 3 als auch die Deckschicht 4 sind im Wesentlichen flächig ausgebildet. Die Folie 1 kann beliebig zugeschnitten werden, um je nach Anwendungsfall eine gute Anformung an eine Knochendefektstelle 2 zu ermöglichen.

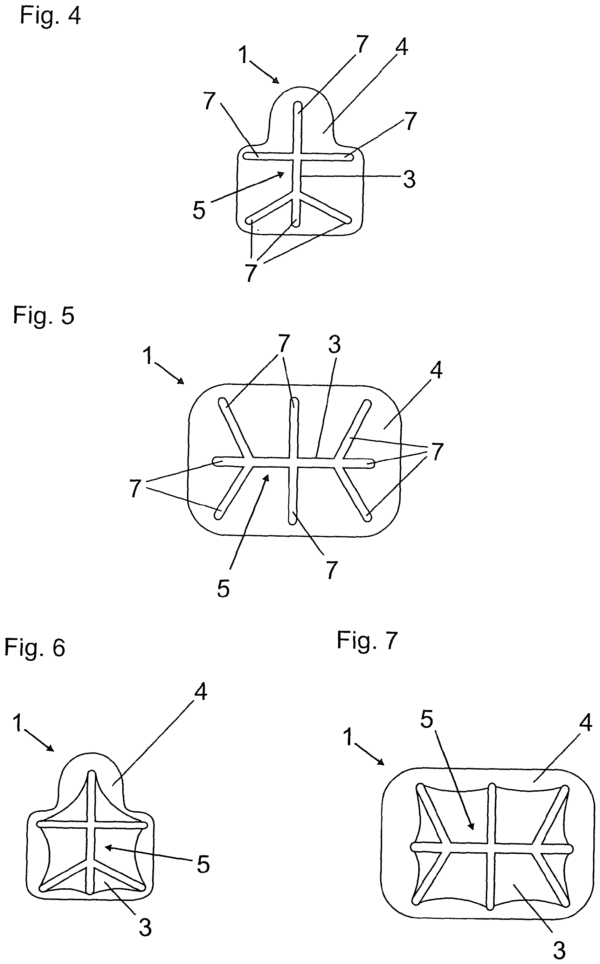

Figur 4 und Figur 5 zeigen zwei weitere Ausführungsformen einer vorgeschlagenen zweischichtigen Folie 1 mit unterschiedlichen äußeren Konturen der Deckschicht 4 und unterschiedlich ausgebildeten Formstrukturen 5 der Formschicht 3.

Figur 6 und Figur 7 zeigen weitere Beispiele von vorgeschlagenen Folien 1, wobei in den hier gezeigten Beispielen die Formschicht 3 jeweils als Gel auf die Deckschicht 4 aufgebracht wurde und in weiterer Folge aushärtete. Die hier gezeigten Formschichten 3 umfassen jeweils eine Formstruktur 5, die beispielsweise dadurch erzielt wurde, dass in den Bereichen der Formstruktur 5 mehr Gel aufgebracht wurde, sodass die Formschichten 3 unterschiedliche Schichtdicken aufweisen. Im Bereich einer Formstruktur 5 weist eine Formschicht 3 eine jeweils stärkere Schichtdicke auf als in den übrigen Bereichen der Formschicht 3.

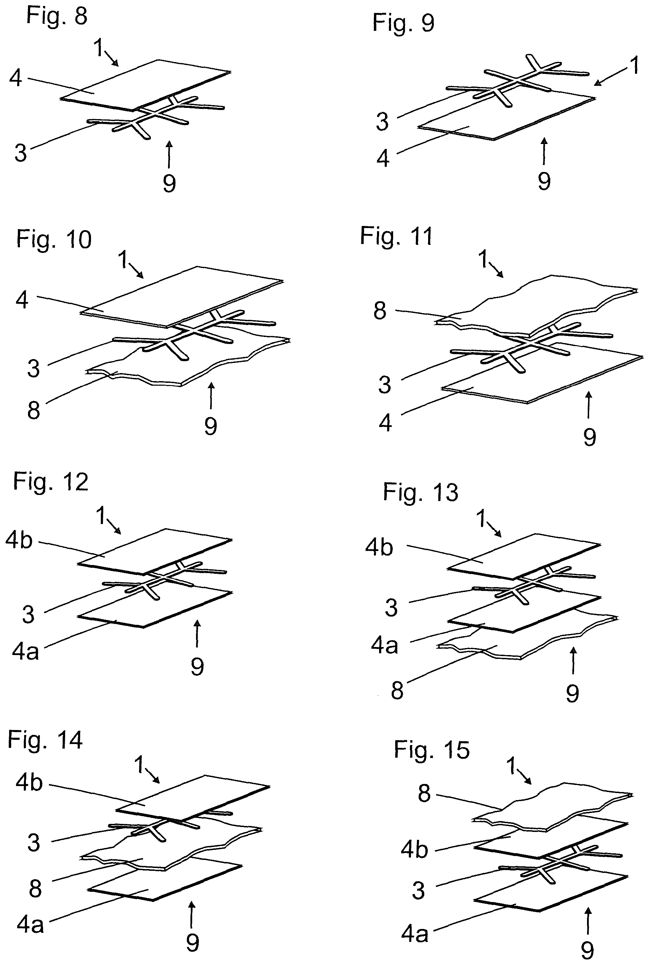

Figur 8 bis Figur 15 zeigen weitere Ausführungsbeispiele einer vorgeschlagenen Folie 1 in jeweils perspektivischer Explosionsdarstellung. Die in den Figuren jeweils nach unten weisende Seite 9 einer Folie 1 ist dabei die einer Knochendefektstelle 2 zuzuwendende Seite 9 der Folie 1.

Die Beispiele der Figur 8 und Figur 9 sind zweischichtig aufgebaut und umfassen jeweils eine Formschicht 3 und eine Deckschicht 4, wobei die Formschicht 3 eine geringere Flächenausdehnung als die Deckschicht 4 einnimmt. Die Beispiele der Figur 10 und Figur 11 sind dreischichtig aufgebaut und umfassen jeweils neben einer Formschicht 3 und einer Deckschicht 4 eine Trägerschicht 8, auf der Substanzen wie beispielsweise Medikamente, Wachstumsfaktoren und/oder sonstige die Heilung und Knochenbildung fördernde und schützende Substanzen aufgebracht sein können.

Die Beispiele der Figuren 12 bis Figur 15 weisen jeweils eine Formschicht 3 und jeweils zwei Deckschichten 4a und 4b auf, wobei die Formschicht 3 eine geringere Flächenausdehnung als die Deckschichten 4a und 4b einnimmt. Die Beispiele der Figur 13 bis Figur 15 weisen jeweils zusätzlich eine Trägerschicht 8 auf, die mit entsprechenden Substanzen (wie oben zu Figur 10 und Figur 11 beschrieben) bestückt sein kann.

Figur 16 zeigt eine Schnittdarstellung eines Kieferknochens 11 mit einer Knochendefektstelle 2. Um einen Knochenaufbau an der Knochendefektstelle 2 zu ermöglichen, wird eine vorgeschlagene vorverbundene mehrschichtige Folie 1 über die Knochendefektstelle 2 entsprechend angeformt und mittels entsprechender Befestigungsvorrichtungen 12 am Kieferknochen 11 verankert. Bei den Befestigungsvorrichtungen 12 kann es sich beispielsweise um resorbierbare Nägel handeln. Im sich bildenden Hohlraum 10 zwischen Folie 1 und Knochendefektstelle 2 bzw. Kieferknochen 11 können Knochenersatzmaterialien und/oder Träger für Medikamente, Wachstumsfaktoren und/oder sonstige die Heilung und Knochenbildung und Ausbildung eines Zahnhalteapparates um natürliche Zähne fördernde und schützende Substanzen enthalten sein, um die Knochenregeneration zu fördern. Nach Anbringung der Folie 1 wird das zuvor entfernte oder zurückgeklappte Zahnfleisch 13 wieder über die Folie 1 gedeckt und entsprechend vernäht. Aufgrund der Resorbierbarkeit der Folie 1 und der Befestigungsvorrichtungen 12 ist keine weitere Operation nötig, um die Folie 1 und/oder die Befestigungsvorrichtungen 12 nach erfolgtem Knochenaufbau wieder zu entfernen.

Figur 17 zeigt eine Knochendefektstelle 2 ähnlich der aus Figur 16, die mit einer vorgeschlagenen Folie 1 abgedeckt ist. In diesem Beispiel wurde in den noch vorhandenen Kieferknochen 11 ein Implantat 14 eingesetzt, dessen freies Ende mit einer Schraube 15 ausgestattet ist. Für eine erleichterte Zugänglichkeit zum Implantat 14 bzw. dessen Schraube 15 kann in diesem Fall vorgesehen sein, dass die Folie 1 bereits mit einem vorab ausgestanzten Loch versehen ist, durch das die Schraube 15 hindurchragen kann.

Figur 18 zeigt ein weiteres Beispiel einer Knochendefektstelle 2, die mit einer vorgeschlagenen Folie 1 abgedeckt ist. Im Kieferknochen 11 ist bereits ein Implantat 14 eingesetzt, an dessen freiem Ende ein Pfosten 16 angeordnet ist. Der Pfosten 16 ragt sowohl durch die Folie 1, als auch durch das Zahnfleisch 13 hindurch, um einen weiteren Zahnaufbau zu erleichtern. Zwischen Pfosten 16 und Folie 1 ist zusätzlich eine Beilagscheibe 18 angeordnet. Durch diese Beilagscheibe 18 kann ein Verschluss bzw. eine Abdichtung der Folie 1 in jenem Bereich der Folie 1 erzielt werden, durch den der Pfosten 16 durch die Folie 1 dringt (Durchdringungsbereich). Dies ist einerseits wichtig, um die Folie 1 gegenüber der Mundhöhle abzudichten und somit die Entstehung von Entzündungen zu hemmen. Andererseits kann damit auch erreicht werden, dass die Folie 1 in genau diesem sensiblen Durchdringungsbereich langsamer resorbiert und damit diesen Bereich besser schützen kann. Die Beilagscheibe 18 kann dabei aus Titan bestehen und das Implantat 14 radial überragen. Die Form der Beilagscheibe 18 kann beispielsweise rund oder oval sein. Die Beilagscheibe 18 kann auch zuschneidbar ausgebildet sein, um je nach Anwendungsfall einen optimalen Verschluss des Durchdringungsbereichs durch die Folie 1 herbeiführen zu können oder um den sensiblen defekten Bereich um das Implantat 14 zu stabilisieren. Dabei kann die Beilagscheibe 18 auch so hergestellt sein, dass die Folie 1 in einer Nut der Beilagscheibe 18 nach Bedarf eingeklemmt und verpresst werden kann, um eine Stabilisierung zu erreichen.

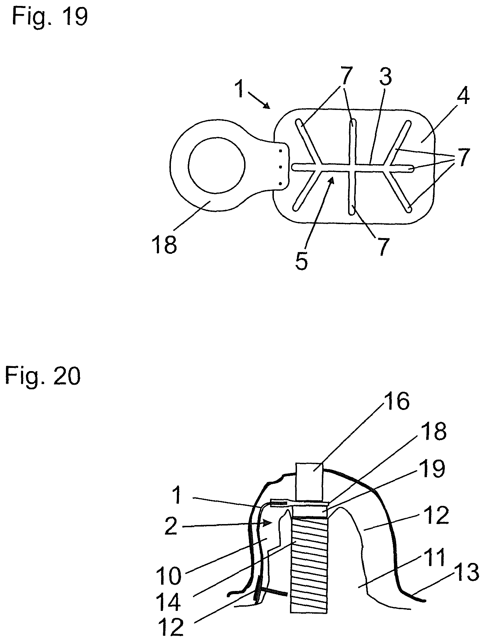

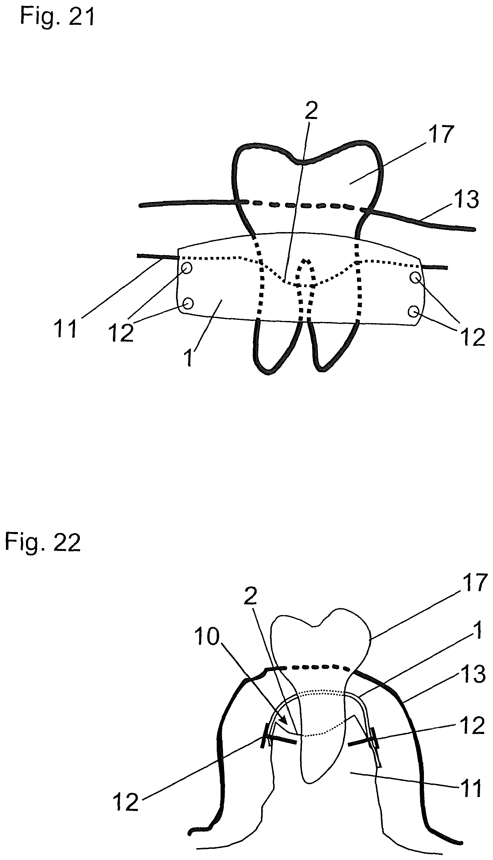

Fig. 19 zeigt eine Folie 1, die in einer Nut einer Beilagscheibe 18 nach eingeklemmt und verpresst ist und Fig. 20 zeigt die Anordnung dieser Folie 1 an einer Knochendefektstelle 2. Im gezeigten Beispiel überragt das obere Ende des Implantats 14 (Implantatkopf) den Kieferknochen 11 nicht, sondern befindet sich unterhalb des Knochenniveaus. Je nachdem, wie tief sich das obere Ende des Implantats 14 im Kieferknochen 11 befindet bzw. wie groß der Niveauunterschied zwischen Implantatkopf und Knochenniveau ist, kann durch verschieden hohe Einsätze 19 ein Ausgleich geschaffen werden, sodass die Folie 1 oder Beilagscheibe 18 ohne Kraterbildung fixiert werden kann. Figur 21 zeigt ein Beispiel einer Knochendefektstelle 2 um einen natürlichen Zahn 17, die mit einer vorgeschlagenen Folie 1 abgedeckt ist. In diesem Beispiel handelt es sich um die Anwendung der vorgeschlagenen Folie 1 zur Abdeckung einer parodontalen Konchendefektstelle 2. Die von der Folie 1 abgedeckten Abschnitte des Kieferknochens 11 und des Zahnes 17 sind dabei strichliert dargestellt.

Figur 22 zeigt eine Schnittdarstellung eines Kieferknochens 11 mit einer Knochendefektstelle 2 um einen natürlichen Zahn 17. Um einen Knochenaufbau und/oder einen Aufbau des Zahnhalteapparates an der Knochendefektstelle 2 zu ermöglichen, wird eine vorgeschlagene vorverbundene mehrschichtige Folie 1 über die Knochendefektstelle 2 und den Zahn 17 entsprechend angeformt und mittels entsprechender Befestigungsvorrichtungen 12 am Kieferknochen 11 verankert. Bei den Befestigungsvorrichtungen 12 kann es sich beispielsweise um resorbierbare Nägel handeln. Im sich bildenden Hohlraum 10 zwischen Folie 1 und Knochendefektstelle 2 bzw. Kieferknochen 11 können Knochenersatzmaterialien und/oder Träger für Medikamente, Wachstumsfaktoren und/oder sonstige die Heilung und Knochenbildung und Ausbildung eines Zahnhalteapparates um natürliche Zähne fördernde und schützende Substanzen enthalten sein, um die Knochenregeneration zu fördern. Nach Anbringung der Folie 1 wird das zuvor entfernte oder zurückgeklappte Zahnfleisch 13 wieder über die Folie 1 gedeckt und entsprechend vernäht. Aufgrund der Resorbierbarkeit der Folie 1 und der Befestigungsvorrichtungen 12 ist keine weitere Operation nötig, um die Folie 1 und/oder die Befestigungsvorrichtungen 12 nach erfolgtem Knochenaufbau wieder zu entfernen.

## Patentansprüche

1. Vorverbundene mehrschichtige Folie (1) zur Abdeckung einer Knochendefektstelle (2), insbesondere im Bereich eines Kieferknochens, wobei die Folie (1) eine formgebende Formschicht (3) zur Anformung der Folie (1) an die Knochendefektstelle (2) und wenigstens eine Deckschicht (4, 4a, 4b) zur Abdeckung der Knochendefektstelle (2) umfasst, wobei die Formschicht (3) und die wenigstens eine Deckschicht (4, 4a, 4b) im Wesentlichen vollständig resorbierbar sind, und die Formschicht (3) zumindest teilweise, vorzugsweise im Wesentlichen vollständig, aus einem bioresorbierbaren Polymermaterial besteht, **dadurch gekennzeichnet, dass** die wenigstens eine Deckschicht (4) im Wesentlichen vollständig aus einem bioresorbierbaren Collagenmaterial besteht.

2. Vorverbundene mehrschichtige Folie (1) nach Anspruch 1, **dadurch gekennzeichnet, dass** die Formschicht (3) und die wenigstens eine Deckschicht (4, 4a, 4b) mechanisch und/oder chemisch miteinander verbunden sind.

3. Vorverbundene mehrschichtige Folie (1) nach Anspruch 1 oder 2, **dadurch gekennzeichnet, dass** die Formschicht (3) und die wenigstens eine Deckschicht (4, 4a, 4b) in unterschiedlichen Zeiträumen im Wesentlichen vollständig resorbierbar sind.

4. Vorverbundene mehrschichtige Folie (1) nach einem der Ansprüche 1 bis 3, **dadurch gekennzeichnet, dass** die Folie (1) insgesamt in einem Zeitraum von etwa 3 bis 12 Monaten, vorzugsweise etwa 4 bis 6 Monaten, im Wesentlichen vollständig resorbierbar ist.

5. Vorverbundene mehrschichtige Folie (1) nach einem der Ansprüche 1 bis 4, **dadurch gekennzeichnet, dass** die Formschicht (3) steifer als die wenigstens eine Deckschicht (4) ausgebildet ist.

6. Vorverbundene mehrschichtige Folie (1) nach einem der Ansprüche 1 bis 5, **dadurch gekennzeichnet, dass** die Formschicht (3), gegebenenfalls zusammen mit der wenigstens einen Deckschicht (4, 4a, 4b), mechanisch und/oder thermisch und/oder chemisch verformbar ausgebildet ist.

7. Vorverbundene mehrschichtige Folie nach einem der Ansprüche 1 bis 6, **dadurch gekennzeichnet, dass** das bioresorbierbare Collagenmaterial Typ-I-Collagen und/oder Typ-III-Collagen umfasst.

8. Vorverbundene mehrschichtige Folie (1) nach einem der Ansprüche 1 bis 7, **dadurch gekennzeichnet, dass** das bioresorbierbare Polymermaterial Milchsäure, vorzugsweise L-Milchsäure, und/oder deren Derivate umfasst, wobei vorzugsweise der Anteil der Milchsäure im bioresorbierbaren Polymermaterial mindestens 70%, vorzugsweise etwa 80% bis 95%, besonders bevorzugt im Wesentlichen etwa 82%, beträgt.

9. Vorverbundene mehrschichtige Folie (1) nach einem der Ansprüche 1 bis 8, **dadurch gekennzeichnet, dass** das bioresorbierbare Polymermaterial Glykolsäure umfasst, wobei vorzugsweise der Anteil der Glykolsäure im bioresorbierbaren Polymermaterial höchstens 30%, vorzugsweise etwa 15% bis 20%, besonders bevorzugt im Wesentlichen etwa 18%, beträgt.

10. Vorverbundene mehrschichtige Folie (1) nach einem der Ansprüche 1 bis 9, **dadurch gekennzeichnet, dass** die Formschicht (3) und die wenigstens eine Deckschicht (4) unterschiedliche Flächenausdehnungen aufweisen, wobei vorzugsweise die Formschicht (3) eine geringere Flächenausdehnung als die wenigstens eine Deckschicht (4) einnimmt.

11. Vorverbundene mehrschichtige Folie (1) nach einem der Ansprüche 1 bis 10, **dadurch gekennzeichnet, dass** die wenigstens eine Deckschicht (4) und/oder die Formschicht (3) im Wesentlichen durchgehend flächig ausgebildet ist bzw. sind.

12. Vorverbundene mehrschichtige Folie (1) nach einem der Ansprüche 1 bis 11, **dadurch gekennzeichnet, dass** die Formschicht (3) zur Anformung an die Knochendefektstelle (2) eine Formstruktur (5) aufweist.

13. Vorverbundene mehrschichtige Folie (1) nach Anspruch 12, **dadurch gekennzeichnet, dass** die Formstruktur (5) wenigstens abschnittsweise einen konvex und/oder konkav gekrümmten Rand (6) und/oder wenigstens abschnittsweise eine konvex und/oder konkav gekrümmte Form aufweist.

14. Vorverbundene mehrschichtige Folie (1) nach Anspruch 12 oder 13, **dadurch gekennzeichnet, dass** die Formstruktur (5) wenigstens ein strebenförmiges Anformelement (7) aufweist.

15. Vorverbundene mehrschichtige Folie (1) nach einem der Ansprüche 12 bis 14, **dadurch gekennzeichnet, dass** die Formstruktur (5) im Wesentlichen gitterförmig ausgebildet ist.

16. Vorverbundene mehrschichtige Folie (1) nach einem der Ansprüche 12 bis 15, **dadurch gekennzeichnet, dass** die Formstruktur (5) durch wenigstens eine Verstärkung der Formschicht (3) ausgebildet ist.

17. Vorverbundene mehrschichtige Folie nach einem der Ansprüche 1 bis 16, **dadurch gekennzeichnet, dass** die Folie (1) eine Trägerschicht (8) für wenigstens eine darauf anzuordnende oder angeordnete Substanz aufweist.

## Claims

1. A pre-bonded multilayer film (1) for covering a bone defect site (2), in particular in the region of a jawbone, wherein the film (1) comprises a shaping molding layer (3) for shaping the film (1) to the bone defect site (2) and at least one cover layer (4, 4a, 4b) for covering the bone defect site (2), wherein the molding layer (3) and the at least one cover layer (4, 4a, 4b) are substantially completely resorbable, and the molding layer (3) at least partially and preferably substantially completely consists of a bioresorbable polymer material, **characterized in that** the at least one cover layer (4) substantially completely consists of a bioresorbable collagen material.

2. A pre-bonded multilayer film (1) as set forth in claim 1 **characterized in that** the molding layer (3) and the at least one cover layer (4, 4a, 4b) are mechanically and/or chemically connected together.

3. A pre-bonded multilayer film (1) as set forth in claim 1 or claim 2 **characterized in that** the molding layer (3) and the at least one cover layer (4, 4a, 4b) are substantially completely resorbable in different periods of time.

4. A pre-bonded multilayer film (1) as set forth in one of claims 1 through 3 **characterized in that** the film (1) is substantially completely resorbable overall in a period of time of between about 3 and 12 months, preferably between about 4 and 6 months.

5. A pre-bonded multilayer film (1) as set forth in one of claims 1 through 4 **characterized in that** the molding layer (3) is stiffer than the at least one cover layer (4).

6. A pre-bonded multilayer film (1) as set forth in one of claims 1 through 5 **characterized in that** the molding layer (3), possibly together with the at least one cover layer (4, 4a, 4b), is adapted to be mechanically and/or thermally and/or chemically deformable.

7. A pre-bonded multilayer film as set forth in one of claims 1 through 6 **characterized in that** the bioresorbable collagen material comprises type-I-collagen and/or type-III-collagen.

8. A pre-bonded multilayer film (1) as set forth in one of claims 1 through 7 **characterized in that** the bioresorbable polymer material comprises lactic acid, preferably L-lactic acid, and/or derivatives thereof, wherein preferably the proportion of lactic acid in the bioresorbable polymer material is at least 70%, preferably between about 80% and 95%, particularly preferably substantially about 82%.

9. A pre-bonded multilayer film (1) as set forth in one of claims 1 through 8 **characterized in that** the bioresorbable polymer material comprises glycolic acid, wherein preferably the proportion of glycolic acid in the bioresorbable polymer material is at most 30%, preferably between about 15% and 20%, particularly preferably substantially about 18%.

10. A pre-bonded multilayer film (1) as set forth in one of claims 1 through 9 **characterized in that** the molding layer (3) and the at least one cover layer (4) have different surface areas, wherein preferably the molding layer (3) involves a smaller surface area than the at least one cover layer (4).

11. A pre-bonded multilayer film (1) as set forth in one of claims 1 through 10 **characterized in that** the at least one cover layer (4) and/or the molding layer (3) is or are substantially flat throughout.

12. A pre-bonded multilayer film (1) as set forth in one of claims 1 through 11 **characterized in that** the molding layer (3) has a shaping structure (5) for shaping molding to the bone defect site (2).

13. A pre-bonded multilayer film (1) as set forth in claim 12 **characterized in that** the shaping structure (5) has at least portion-wise a convexly and/or concavely curved edge (6) and/or at least portion-wise a convexly and/or concavely curved shape.

14. A pre-bonded multilayer film (1) as set forth in claim 12 or 13 **characterized in that** the shaping structure (5) has at least one strut-shaped shaping molding element (7).

15. A pre-bonded multilayer film (1) as set forth in one of claims 12 through 14 **characterized in that** the shaping structure (5) is substantially grid-shaped.

16. A pre-bonded multilayer film (1) as set forth in one of claims 12 through 15 **characterized in that** the shaping structure (5) is provided by at least one reinforcement of the molding layer (3).

17. A pre-bonded multilayer film as set forth in one of claims 1 through 16 **characterized in that** the film (1) has a carrier layer (8) for at least one substance which is arranged or which is to be arranged thereon.

## Revendications

1. Feuille (1) multicouche préassemblée destinée à recouvrir un défaut osseux (2), en particulier dans la zone d'un os de la mâchoire, la feuille (1) comprenant une couche de forme (3) de mise en forme destinée à conformer la feuille (1) sur le défaut osseux (2) et au moins une couche de recouvrement (4, 4a, 4b) destinée à recouvrir le défaut osseux (2), la couche de forme (3) et l'au moins une couche de recouvrement (4, 4a, 4b) étant résorbables essentiellement complètement, et la couche de forme (3) se composant au moins partiellement, de préférence essentiellement complètement, d'un matériau polymère biorésorbable, **caractérisée en ce que** l'au moins une couche de recouvrement (4) se compose essentiellement complètement d'un matériau collagène biorésorbable.

2. Feuille (1) multicouche préassemblée selon la revendication 1, **caractérisée en ce que** la couche de forme (3) et l'au moins une couche de recouvrement (4, 4a, 4b) sont raccordées l'une à l'autre mécaniquement et/ou chimiquement.

3. Feuille (1) multicouche préassemblée selon la revendication 1 ou 2, **caractérisée en ce que** la couche de forme (3) et l'au moins une couche de recouvrement (4, 4a, 4b) sont résorbables essentiellement complètement dans des périodes de temps différentes.

4. Feuille (1) multicouche préassemblée selon l'une des revendications 1 à 3, **caractérisée en ce que** la feuille (1) au total est résorbable essentiellement complètement dans une période d'environ 3 à 12 mois, de préférence d'environ 4 à 6 mois, au total.

5. Feuille (1) multicouche préassemblée selon l'une des revendications 1 à 4, **caractérisée en ce que** la couche de forme (3) est constituée de façon plus rigide que l'au moins une couche de recouvrement (4).

6. Feuille (1) multicouche préassemblée selon l'une des revendications 1 à 5, **caractérisée en ce que** la couche de forme (3), éventuellement conjointement avec l'au moins une couche de recouvrement (4, 4a, 4b), est constituée de façon mécaniquement et/ou thermiquement et/ou chimiquement déformable.

7. Feuille (1) multicouche préassemblée selon l'une des revendications 1 à 6, **caractérisée en ce que** le matériau collagène biorésorbable comprend du collagène de type I et/ou du collagène de type III.

8. Feuille (1) multicouche préassemblée selon l'une des revendications 1 à 7, **caractérisée en ce que** le matériau polymère biorésorbable comprend de l'acide lactique, de préférence de l'acide lactique L, et/ou ses dérivés, la proportion de l'acide lactique dans le matériau polymère biorésorbable étant de préférence d'au moins 70 %, de préférence d'environ 80 % à 95 %, de façon particulièrement préférée essentiellement d'environ 82 %.

9. Feuille (1) multicouche préassemblée selon l'une des revendications 1 à 8, **caractérisée en ce que** le matériau polymère biorésorbable comprend de l'acide glycolique, la proportion de l'acide glycolique dans le matériau polymère biorésorbable étant de préférence de 30 % maximum, de préférence d'environ 15 % à 20 %, de façon particulièrement préférée essentiellement d'environ 18 %.

10. Feuille (1) multicouche préassemblée selon l'une des revendications 1 à 9, **caractérisée en ce que** la couche de forme (3) et l'au moins une couche de recouvrement (4) comportent des extensions de surface différentes, la couche de forme (3) occupant de préférence une extension de surface plus faible que l'au moins une couche de recouvrement (4).

11. Feuille (1) multicouche préassemblée selon l'une des revendications 1 à 10, **caractérisée en ce que** l'au moins une couche de recouvrement (4) et/ou la couche de forme (3) est ou respectivement sont constituée(s) essentiellement à plat de façon continue.

12. Feuille (1) multicouche préassemblée selon l'une des revendications 1 à 11, **caractérisée en ce que** la couche de forme (3) présente une structure de forme (5) pour la conformation sur le défaut osseux (2).

13. Feuille (1) multicouche préassemblée selon la revendication 12, **caractérisée en ce que** la structure de forme (5) comporte au moins par tronçons un bord (6) courbé de façon convexe et/ou concave et/ou au moins par tronçons une forme courbée de façon convexe et/ou concave.

14. Feuille (1) multicouche préassemblée selon la revendication 12 ou 13, **caractérisée en ce que** la structure de forme (5) comporte au moins un élément de conformation (7) en forme d'entretoise.

15. Feuille (1) multicouche préassemblée selon l'une des revendications 12 à 14, **caractérisée en ce que** la structure de forme (5) est constituée essentiellement en forme de grille.

16. Feuille (1) multicouche préassemblée selon l'une des revendications 12 à 15, **caractérisée en ce que** la structure de forme (5) est constituée d'au moins un renforcement de la couche de forme (3).

17. Feuille (1) multicouche préassemblée selon l'une des revendications 1 à 16, **caractérisée en ce que** la feuille (1) comporte une couche de support (8) pour au moins une substance à disposer dessus ou disposée dessus.
